# FASCICULE DE BREVET EUROPEEN

(11) **EP 3 825 677 B1**
(45) Date de publication et mention de la délivrance du brevet: **23.04.2025**
(21) Numéro de dépôt: 20209075.9
(22) Date de dépôt: 20.11.2020
(51) Int. Cl.: G01N 1/28, B01L 3/00, C12M 3/06, G01N 1/40

(54) **DISPOSITIF DE LOCALISATION D'OBJETS BIOLOGIQUES**
VORRICHTUNG ZUR LOKALISIERUNG VON BIOLOGISCHEN OBJEKTEN
DEVICE FOR LOCATING BIOLOGICAL OBJECTS

(30) Priorité: 21.11.2019 FR 1912994
(43) Date de publication de la demande: 26.05.2021
(73) Titulaire: Commissariat à l'Energie Atomique et aux Energies Alternatives, 75015 Paris (FR); Institut National de la Santé et de la Recherche Médicale, 75013 Paris (FR)
(72) Inventeur: CASSET, Fabrice, 38054 Grenoble Cedex 09 (FR); MILLET, Arnaud, 38340 Voreppe (FR); NEEF, Baptiste, 38054 Grenoble Cedex 09 (FR)
(74) Mandataire: Brevalex

(56) Documents cités:
- EP-A1- 1 734 110
- FR-A1- 3 032 974
- FR-A1- 3 043 093
- US-A1- 2012 003 709
- BAPTISTE NEFF ET AL: "Development of a MEMS Plate Based on Thin-Film Piezoelectric AlN Actuators for Biological Applications", PROCEEDINGS, vol. 1, no. 4, 9 August 2017 (2017-08-09), pages 386, XP055714785, DOI: 10.3390/proceedings1040386

## Description

### DOMAINE TECHNIQUE ET ÉTAT DE LA TECHNIQUE ANTÉRIEURE

La présente invention se rapporte à un dispositif de localisation d'objets biologiques sur une surface, notamment en vue d'une étude de la migration cellulaire.

L'étude de la migration cellulaire est un enjeu majeur de l'étude du comportement cellulaire dans un contexte physiologique ou pathologique. Une des méthodes utilisées pour étudier la migration cellulaire in vitro comporte la réalisation d'une ou plusieurs « cicatrices » dans un tapis cellulaire et l'analyse de la fermeture de la ou des cicatrices. Cette fermeture est la résultante directe de la migration des cellules présentes. Une des techniques pour former la cicatrice utilise un stylet ou une pipette, la formation de la cicatrice est donc peu contrôlée et son niveau de reproductibilité est faible. Une autre technique met en œuvre des inserts, mais les tailles disponibles pour les inserts et la nécessité de coller ceux-ci peuvent interférer avec le test. D'autres techniques utilisent une fonctionnalisation chimique qui permet d'étudier l'interaction entre les cellules et leur substrat mais impliquent de tenir compte de la chimie utilisée.

Le document FR3043093A1 présente un dispositif MEMS de traitement de cellules biologiques, tandis que le document FR3032974A1 prévoit un dispositif MEMS de manipulation de cellules biologiques.

L'article "Development of a MEMS Plate Based on Thin-Film Piezoelectric AIN Actuators for Biological Applications", de Neff et al., Eurosensors 2017 Conference, présente le développement d'un système de laboratoire sur puce basé sur l'utilisation de vibrations locales pour stimuler mécaniquement des éléments biologiques.

### EXPOSÉ DE L'INVENTION

C'est par conséquent un but de la présente invention d'offrir un dispositif de localisation d'objets biologiques sur une surface de manière contrôlée et répétable.

Le but énoncé ci-dessus est atteint par un dispositif de localisation cellulaire comportant une plaque suspendue, dont une face présente des propriétés d'adhérence par rapport aux cellules, des moyens configurés pour actionner la plaque selon un mode de vibration stationnaire à une longueur d'onde supérieure à la taille des objets biologiques, par exemple la longueur d'onde est supérieure à 400 nm.

Les inventeurs ont constaté que les objets biologiques se répartissaient de sorte à former une zone dénuée de cellule, par exemple dans une zone de ventre de la déformée de la plaque actionnée selon un mode stationnaire.

Cette méthode permet donc de réaliser des « cicatrices » ou zones d'exclusion dans des zones déterminées, i.e. par exemple les zones de ventre, et de largeur égale à environ 1/4 de la longueur d'onde. En outre ces cicatrices sont répétables, ce qui permet de réaliser des études réellement comparatives entre différentes populations de cellules.

L'étude de la migration des cellules biologiques est donc rendue plus fiable.

En d'autres termes, à l'aide de la génération d'ondes mécaniques dans un élément vibrant provoquant l'apparition de ventres et de creux, les objets adhérents sont localisés spatialement sur l'élément vibrant. Dans le cas des cellules biologiques, cela permet de définir des zones d'exclusion de l'adhésion cellulaire pour créer par exemple des « cicatrices », qui peuvent ensuite être envahies par les cellules après arrêt des vibrations et permettre le suivi quantitatif de la migration.

Les cellules ne sont pas « éjectées » de la zone d'exclusion de la surface de la plaque, mais elles sont localisées dans le liquide avant leur sédimentation sur la plaque et leur adhérence à celle-ci, de sorte à ne pas adhérer dans la zone d'exclusion de la sédimentation.

Ce dispositif est avantageusement compatible avec la culture de cellules de mammifère. Ce dispositif est particulièrement adapté à la quantification de l'impact de molécules thérapeutiques ciblant le cytosquelette pour l'inhibition de l'invasion métastatique dans le cancer.

De manière avantageuse, le dispositif est au moins en partie optiquement transparent pour permettre l'observation des cellules en cours de migration et pour étudier le comportement migratoire des cellules.

Dans un mode préféré, la plaque est simplement supportée dans la cavité fluidique du dispositif, ce qui permet la formation de cicatrices de largeur homogène sur toute la surface de la plaque évitant les effets de bords résultant d'un encastrement.

La présente invention a alors pour objet un dispositif de localisation d'objets biologiques d'au moins une taille donnée comportant une cavité fluidique, des moyens de connexion fluidiques configurés pour permettre d'introduire du liquide et des objets dans la cavité fluidique, une plaque munie d'une surface d'accueil présentant des propriétés d'adhérence vis-à-vis des objets biologiques, ladite plaque étant suspendue dans la cavité fluidique, des moyens pour mettre en vibration la plaque selon au moins un mode stationnaire comportant au moins un actionneur destiné à agir sur la plaque et une unité de commande configurée pour commander les actionneurs de sorte que la plaque vibre selon un mode stationnaire de longueur d'onde λ comprise entre 3 fois et 20 fois la taille donnée des objets biologiques.

De préférence, le au moins un actionneur est disposé par rapport à la plaque au niveau d'un ventre de la déformée de la plaque en mode stationnaire.

Dans un exemple de réalisation, la plaque est simplement supportée. Par exemple, le dispositif de localisation peut comporter des supports supportant la plaque suivant une liaison simplement supportée. De préférence, les supports sont en contact avec la plaque au niveau d'au moins certains nœuds de la déformée de la plaque dans ledit mode stationnaire.

### Dans un autre exemple de réalisation, la plaque est maintenue par encastrement

Le au moins un actionneur peut être un actionneur piézoélectrique fixé à la plaque.

Dans un exemple, au moins une partie de la cavité fluidique est transparente optiquement.

La présente invention a également pour objet un système d'étude de la migration des cellules comportant un dispositif de localisation dans lequel moins une partie de la cavité fluidique est transparente optiquement, et des moyens d'observation des objets biologiques sur la plaque.

La présente invention a également pour objet procédé de localisation d'objets biologiques selon un motif donné mettant en œuvre un dispositif selon l'invention, comportant :
- la mise en place de liquide dans la cavité fluidique au moins sur une partie de la surface d'accueil,
- l'injection d'objets biologiques dans ledit liquide,
- activation du au moins un actionneur de sorte que la plaque vibre selon un mode stationnaire choisi pour obtenir le motif donné,
- arrêt du au moins un actionneur et sédimentation des objets biologiques sur la surface d'accueil selon le motif donné.

Dans un exemple de fonctionnement, le liquide entoure la plaque. Dans un autre exemple de fonctionnement, le liquide est déposé uniquement sur la surface d'accueil.

La présente invention a également pour objet un procédé d'analyse de la migration de cellules biologiques mettant en œuvre le procédé de localisation selon l'invention, les objets biologiques étant des cellules, ledit procédé d'analyse comportant, après la sédimentation de cellules biologiques sur la surface d'accueil formant au moins une cicatrice, l'observation de la migration des cellules biologiques.

### BRÈVE DESCRIPTION DES DESSINS

La présente invention sera mieux comprise sur la base de la description qui va suivre et des dessins en annexe sur lesquels:
La figure 1 est une représentation schématique d'une vue de côté d'un exemple de dispositif de localisation cellulaire.
La figure 2A est une vue en perspective d'une plaque mise en œuvre dans le dispositif de la figure 1 actionnée selon un mode de Lamb.
La figure 2B est une vue de côté de la plaque de figure 2A.
La figure 3 est une vue de côté d'un autre exemple d'un dispositif de localisation dans lequel la plaque est encastrée.
La figure 4A est une représentation de la déformée d'une plaque circulaire encastrée actionnée selon un mode stationnaire.
La figure 4B est une représentation de la déformée d'une plaque rectangulaire encastrée actionnée selon un mode stationnaire.
La figure 5 est une vue en perspective d'une plaque mise en œuvre dans le dispositif de la figure 1 montrant les rangées d'actionneurs.
La figure 6A est une image des cicatrices obtenues grâce au dispositif de localisation.
La figure 6B est une image d'autres cicatrices obtenues grâce au dispositif de localisation.
Les figures 7A, 7B, 7C et 7D sont des représentations schématiques d'étapes de réalisation selon un exemple de réalisation d'une plaque mise en œuvre dans un dispositif de localisation.
La figure 8 représente différents motifs de localisation cellulaire obtenus par le dispositif de localisation.
La figure 9A est une représentation schématique de la plaque lors d'un procédé de localisation par immersion.
La figure 9B est une représentation schématique de la plaque lors d'un procédé de localisation par dépôt d'un volume de liquide sur la plaque.
La figure 10 est une représentation de la déformée d'une plaque rectangulaire actionnée selon un autre mode d'actionnement stationnaire.

### EXPOSÉ DÉTAILLÉ DE MODES DE RÉALISATION PARTICULIERS

La présente invention est particulièrement adaptée à la localisation de cellules biologiques, la description décrira principalement l'application aux cellules biologiques. Mais l'invention permet la localisation de tout objet biologique apte à adhérer à la surface d'une plaque du dispositif. La taille des objets est comprise entre 1 µm et une centaine de µm.

Par exemple il peut s'agir de globules blancs dont la tailles est approximativement comprise entre 7 µm et 18µm, de cellules tumorales circulantes dont la taille est d'environ 10 µm, de globules rouges, dont la taille est comprise entre 6 µm et 8µm environ, de bactéries dont la taille est de l'ordre de 2 µm. La taille des cellules adhérentes peut aller jusqu'à la centaine de micromètres.

La taille des objets est la dimension la plus grande dans le plan. Lorsque l'objet est de forme sensiblement circulaire, sa taille correspond à son diamètre, et lorsqu'il est de forme allongée, sa taille correspond à sa dimension dans la direction allongée.

Le dispositif selon l'invention, particulièrement dans la réalisation de cicatrices, permet de réaliser des zones d'exclusion de largeur supérieure à la taille d'une cellule, de préférence de largeur égale à au moins 100 µm, soit de l'ordre de 10 fois la taille d'une cellule.

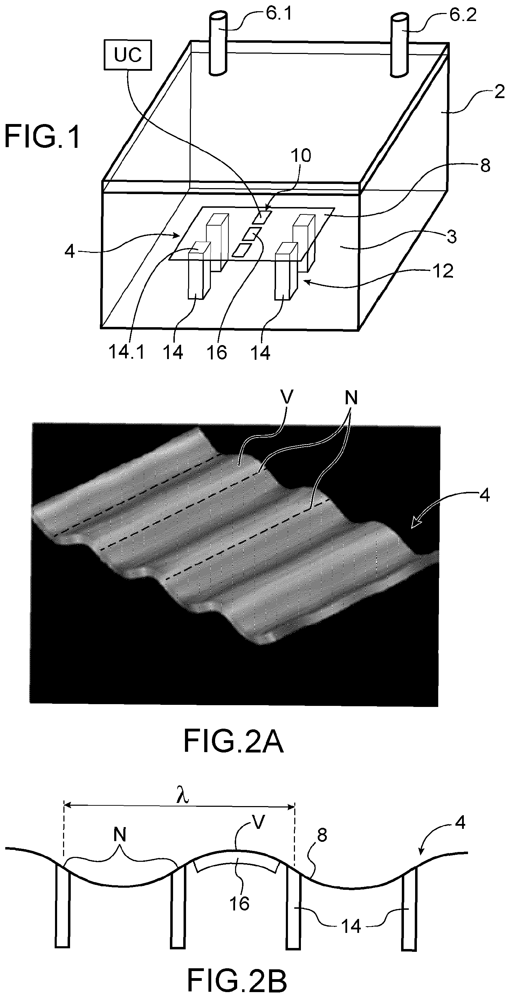

Sur la figure 1, on peut voir une représentation schématique d'un dispositif de localisation de cellules.

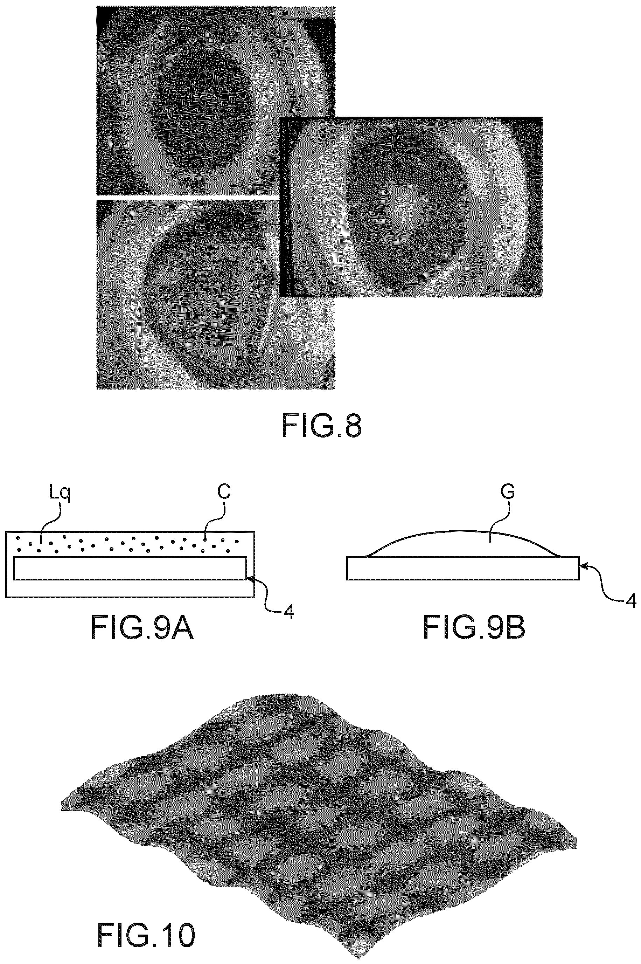

Le dispositif comporte un boîtier 2 délimitant une cavité fluidique 3, dans laquelle est suspendue une plaque 4 de forme rectangulaire, au moins une entrée d'accès à l'intérieur du boîtier soit pour alimenter en liquide l'intérieur du boîtier et immerger la plaque (figure 9A), soit pour déposer du liquide uniquement sur la plaque (figure 9B). Dans l'exemple représenté, le dispositif comporte une entrée d'alimentation 6.1 en liquide et une sortie d'évacuation 6.2 en liquide.

La plaque 4 comporte une face d'accueil 8 sur laquelle les cellules sont destinées à adhérer.

La plaque 4 a par exemple des dimensions comprises entre quelques centaines de µm et quelques centimètres. L'épaisseur de la plaque est par exemple comprise entre 100µm et quelques mm.

La plaque 4 est par exemple en verre, en silicium ou tout matériau semiconducteur. Les propriétés mécaniques de la plaque ont une influence sur l'adhésion des cellules. Une cellule adhérente crée une matrice d'adhésions focales avec la surface qui est nécessaire à l'organisation de son architecture cellulaire. Les forces d'adhésion des cellules ont des valeurs comprises entre de l'ordre de la dizaine de nanonewtons et la centaine de nanonewtons. Ces forces peuvent être caractérisées par l'utilisation de pince optique ou magnétique ainsi que par des microplaques.

Le dispositif comporte également des moyens 10 configurés pour mettre en vibration la plaque 4 selon un mode d'onde stationnaire présentant une longueur d'onde supérieure à la taille des cellules.

La plaque actionnée selon un mode stationnaire va se déformer et présenter des zones se déplaçant avec une amplitude maximale, désignées « ventres », et des zones fixes désignées « nœuds ». Les nœuds restent dans le plan de la plaque, le plan de la plaque contenant la plaque au repos.

Les moyens 10 comportent des actionneurs aptes à exercer une action sur la plaque pour la mettre en vibration.

Dans l'exemple représenté et de manière préférée, la plaque 4 est simplement supportée par une structure support 12 à l'intérieur du boîtier. Dans l'exemple représenté, la structure support 12 comporte des colonnes 14 reposant sur le fond du boîtier et la plaque est déposée sur les extrémités libres 14.1 libres des colonnes. La liaison « simplement supportée » permet d'obtenir une déformée plus homogène, jusqu'au bord de la plaque.

De manière avantageuse, les extrémités libres 14.1 présentent un traitement légèrement adhésif pour assurer un maintien de la plaque dans le plan et éviter un glissement de la plaque.

Le positionnement relatif des colonnes 14 et de la plaque 4 est choisi de sorte que le contact entre les colonnes et la plaque se fasse à des nœuds de vibration, i.e. au niveau des zones fixes. Dans l'exemple représenté, quatre colonnes sont mises en œuvre.

Les moyens 10 comportent des actionneurs 16 pour générer la mise en vibration de la plaque selon un mode stationnaire. Les actionneurs 16 sont disposés par rapport à la plaque de sorte à être situés au niveau d'au moins un ventre de la déformée de la plaque dans le mode stationnaire à actionner.

Par exemple les actionneurs sont des actionneurs ferroélectriques, par exemple mettant en œuvre du PZT (Titano-Zirconate de Plomb). En variante les actionneurs sont des actionneurs piézoélectriques, électrostatiques, magnétiques ou thermiques.

Les actionneurs sont fixés à la plaque, par exemple sur la face opposée à la face d'accueil. En variante, ils sont fixés sur la face d'accueil.

Les actionneurs sont commandés par une unité de commande UC, par exemple intégrée à un ordinateur, et comportant par exemple les conditions de fonctionnement des actionneurs de sorte à actionner la plaque suivant différents modes en fonction par exemple des cellules à étudier.

Sur la figure 2A, on peut voir représentée une plaque actionnée selon un mode de Lamb, avec les ventres V et les nœuds N. Les actionneurs sont répartis sous forme de bande dans la direction des ventres V. Dans cet exemple la direction des bandes est la direction de la largeur de la plaque.

Sur la figure 2B, on peut voir, vue de côté, la déformée de la plaque 4 et les colonnes 14.

La longueur d'onde de la déformée est désignée λ et couvre un ventre et un creux. La longueur d'onde de l'onde stationnaire de la déformée de la plaque est comprise entre 3 à 20 fois la taille de la cellule, préférentiellement elle est égale à 10 fois la taille de la cellule. De préférence, les actionneurs présentent une largeur sensiblement égale à la taille d'un ventre.

L'amplitude du mode de résonance est suffisante pour déplacer le liquide et donc les cellules en suspension dans celui-ci. Typiquement l'amplitude est comprise entre quelques dizaines de nm et quelques micromètres.

Dans un exemple, les moyens sont adaptés pour actionner la plaque en utilisant tous les actionneurs de sorte que les ventres soient toujours aux mêmes emplacements.

Dans un autre exemple avantageux, les moyens 10 peuvent activer tout ou partie des actionneurs, offrant ainsi une plus grande liberté dans le choix de l'actionnement de la plaque.

L'emplacement des actionneurs et leur taille peuvent être déterminés en utilisant un logiciel de calcul par éléments finis, tel que COMSOL^{®}, ANSYS^{®} ou COVENTOR^{®}, à partir de la déformée dans le mode de vibration choisi. La fréquence de résonance de ce mode et l'amplitude peuvent également être déterminées par simulation par éléments finis et/ou par calcul analytique. La fréquence et l'amplitude sont fonction de la tension appliquée aux actionneurs.

La détermination des actionneurs peut être réalisée comme cela est expliqué dans le document Casset et al, « Low voltage actuated plate for haptic applications with PZT thin-film », Proceedings of Transducers 2013*.*

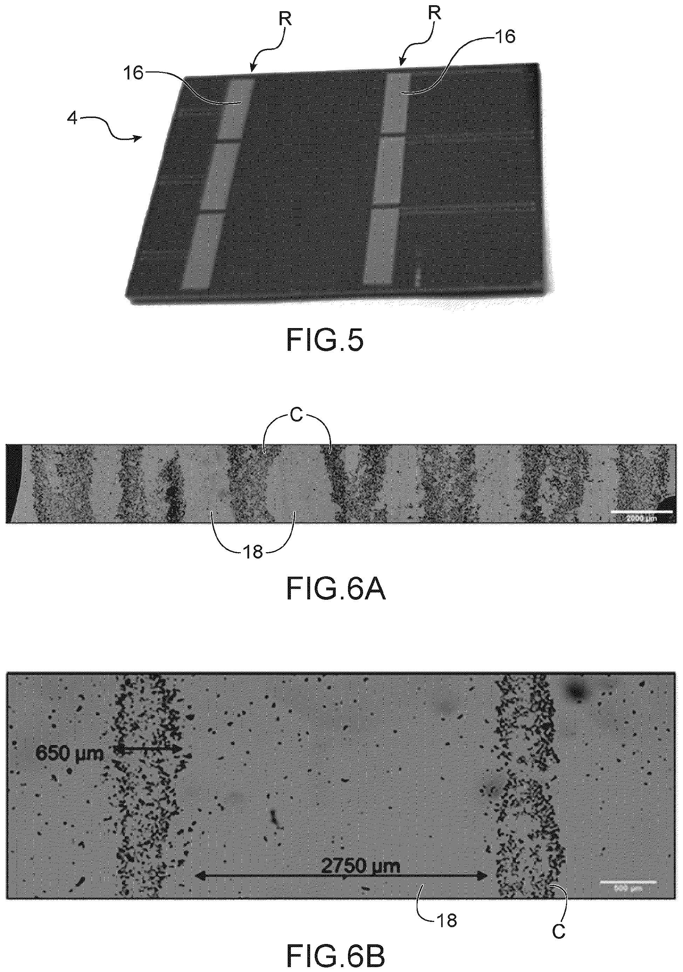

Sur la figure 5, on peut voir un exemple de plaques avec deux rangées R d'actionneurs piézoélectriques 16. Chaque rangée comporte trois actionneurs et est située au niveau d'un ventre de la déformée ou des déformés souhaitées. Dans cet exemple la plaque mesure 30 mm de long par 20 mm de large et les actionneurs sont en PZT.

Un seul actionneur par rangée est envisageable.

Il n'est pas requis que des actionneurs soient positionnés à chaque ventre de la déformée.

Un exemple d'un procédé de formation d'une cicatrice mettant en œuvre le dispositif décrit ci-dessus va maintenant être décrit.

On considère le cas où la plaque 4 est immergée dans un liquide (figure 9A).

Le liquide Lq, par exemple un liquide adapté à la vie des cellules C, est injecté dans la cavité fluidique 3 noyant la plaque 4. Le liquide utilisé forme un milieu de culture biologique standard pour la culture cellulaire, par exemple il s'agit du milieu minimal essentiel de Eagle modifié de Vogt/Dulbecco ou DMEM Dulbecco/Vogt modified Eagle's minimal essential medium en terminologie anglo-saxonne ou du RPMI 1640 (Roswell Park Memorial Institute medium 1640).

Ensuite, les cellules sont injectées dans le liquide, par exemple au moyen d'une pipette, et se répartissent de manière homogène dans le liquide.

L'unité de commande UC active les actionneurs 16 pour mettre la plaque 4 en vibration suivant le mode et la fréquence choisis, établis préalablement pour générer des cicatrices de largeur donnée à un ou des emplacements donnés. L'activation des actionneurs a lieu pendant la sédimentation et avant l'adhérence des cellules sur la surface d'accueil. Les actionneurs peuvent être activés avant l'injection des cellules.

Les cellules C se répartissent alors au-dessus de la surface d'accueil 8 et plus particulièrement au-dessus des ventres de vibration de la plaque 4 et s'éloignent des nœuds de vibrations. Les cellules viennent sédimenter sur la surface d'accueil 8 et adhèrent à celle-ci. En variante, les actionneurs peuvent être activés jusqu'à la sédimentation de toutes les cellules ou être arrêtés au cours de la sédimentation.

Sur la figure 6A, on peut voir un exemple de répartition de cellules obtenu grâce au dispositif selon l'invention. Dans cet exemple, 7 cicatrices 18 de largeur moyenne de 1787 µm sont formées. La distance moyenne entre les cicatrices est de 1380 µm.

Sur la figure 6B, on peut voir un autre exemple de répartition obtenu grâce au dispositif. Les cicatrices 18 ont une largeur de 2750 µm et les cellules sont réparties entres les cicatrices sur une largeur de 650 µm.

Une étude de la migration des cellules peut ensuite être réalisée. De manière avantageuse le boîtier et la plaque sont réalisés dans des matériaux transparents optiquement permettant de visualiser les cellules lors de leur migration, par exemple par une observation au moyen d'un microscope. Des photos et/ou un film peuvent être avantageusement réalisés au cours de la migration.

La vitesse de migration dépend des cellules et du substrat pour des cicatrices d'une largeur de 100 µm à 200 µm, la durée de migration est de l'ordre d'une journée voire quelques jours.

Selon une variante schématisée sur la figure 9B, un volume G de liquide par exemple de l'ordre 2 mL est déposé uniquement sur la plaque 4 et les cellules C sont ensuite injectées dans ce volume de liquide. Le volume peut ne couvrir qu'une partie de la surface d'accueil et permettre donc de limiter la zone de localisation des cellules sur la surface d'accueil. La mouillabilité de la surface d'accueil de la plaque est prise en compte afin de pouvoir déposer un volume de liquide sur celle-ci.

Le mouvement de la plaque génère des forces à l'intérieur de celui-ci.

Le fait de déposer le volume de liquide directement sur la plaque permet de mieux contrôler l'endroit où sont déposées les cellules. Dans le cas d'une immersion totale, les cellules sont déposées sur toute la plaque.

Les conditions extérieures pour la migration peuvent être modifiées, par exemple la température. Le dispositif peut alors comporter des moyens de chauffage contrôlables. Des espèces chimiques peuvent être introduites en même temps que les cellules.

A titre d'illustration, nous allons donner des exemples de dimensionnement des plaques et des actionneurs pour réaliser des cicatrices de l'ordre de 100 µm à 2000 µm de large. On considère une plaque de verre de 30×20mm².

Les cicatrices se forment au niveau du maximum de déplacement de la structure vibrante et ont une largeur d'environ % de la longueur d'onde définie par les caractéristiques de la plaque et des actionneurs.

Par calcul par éléments finis, on réalise une analyse modale de la plaque pour obtenir la déformée du mode considéré, par exemple le mode de Lamb représenté sur la figure 2A.

Selon un premier exemple, on souhaite générer des cicatrices de l'ordre de 2000 µm de large, cela implique une déformée de longueur d'onde d'environ 8000 µm, environ 4 périodes sur une plaque de 30 mm de long

Chaque période fait 7500 µm. La zone correspondant au maximum de déformation de la plaque, de l'ordre du quart de la période, a pour ce mode à 175,4kHz, une largeur de l'ordre de 1875µm, ce qui est conforme à l'objectif de cicatrice d'environ 2000 µm.

Les actionneurs favorisant ce mode et la génération de cicatrices d'environ 2000 µm, seront positionnés sur des zones de déplacement maximal de la plaque. Par exemple, une ligne d'actionneurs 16 en PZT, de l'ordre de 1500 µm de largeur, positionnée à 2600 µm d'un bord de la plaque et parallèlement à celui-ci, et une seconde ligne d'actionneurs 16 espacée de la première colonne d'actionneurs, d'une période soit 7500µm.

Selon un deuxième exemple, on souhaite former une cicatrice de largeur inférieure à 1000 µm, nous pouvons choisir un mode avec 8 périodes au lieu de 4. La cicatrice a une largeur de 937 µm.

On peut alors positionner deux lignes d'actionneurs 16 d'une largeur de 750 µm, séparées d'une période, soit 3750 µm.

Comme cela a été mentionné ci-dessus, des actionneurs au niveau de chaque ventre ne sont pas requis, cela est montré par les exemples ci-dessus. Le premier exemple met en œuvre deux lignes d'actionneurs alors que quatre ventres sont générés, et le deuxième exemple met en œuvre deux lignes d'actionneurs alors que huit ventres sont générés.

D'autres modes d'actionnement de la plaque sont envisageables. Par exemple, on peut choisir un mode d'actionnement en forme de damier comme cela est représenté sur la figure 10. Dans ce cas les actionneurs peuvent être répartis de sorte à former un quadrillage.

Toute autre forme de plaque est envisageable, par exemple une forme de poutre, i.e. très longue par rapport à sa largeur, une forme de disque.

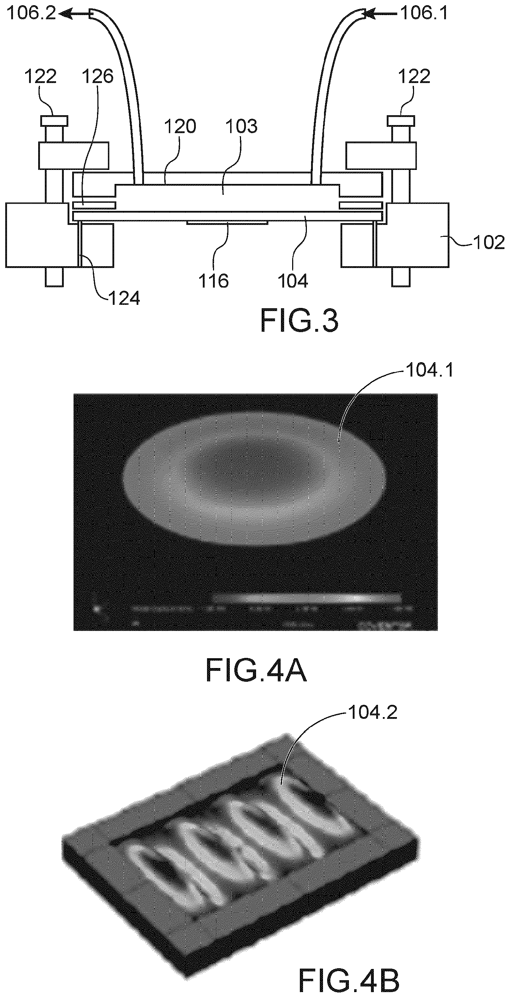

Selon une variante représentée sur la figure 3, la plaque 104 est encastrée au support. Dans cette variante, la plaque 104 est encastrée entre un support 102 et un capot 120. La plaque 104 forme le fond de la cavité fluidique 103. Dans cette variante, le capot 120 est fixé au support 102 par des vis 122. Les connexions fluidiques 106.1, 106.2 sont réalisées dans le capot.

La connexion électrique 124 entre les actionneurs 116 et l'unité de commande UC est réalisée à travers le support 102.

De manière avantageuse, un joint 126 est interposé entre la plaque 104 et le capot 120.

De préférence le capot et éventuellement la plaque sont transparents pour permettre une observation in situ des cellules.

Sur les figures 4A et 4B, on peut voir des représentations de la déformée des plaques en forme de disque 104.1 et de forme rectangulaire 104.2 encastrées au support. Dans le cas de la plaque rectangulaire 104.2 les zones de ventre présentent une largeur moins uniforme sur toute la largeur de la plaque, par rapport à la plaque 4 simplement supportée par les colonnes.

Le dispositif selon l'invention permet de localiser les cellules sur une surface possédant des propriétés adhérentes par rapport aux cellules suivant tout motif qui peut être obtenu par des modes d'actionnement de la plaque. Sur la figure 8, on peut voir des exemples de motifs de cellules obtenus grâce au dispositif de l'invention par la vibration de plusieurs modes de résonance d'une membrane circulaire. De tels motifs permettent de réaliser des cicatrices, ou de localiser des cellules à un endroit donné pour qu'elles y subissent un ou plusieurs traitements particuliers par rapport à d'autres cellules localisées à un autre endroit qui subissent un ou plusieurs autres traitements particuliers ou aucun traitement.

On peut envisager de localiser différentes cellules successivement selon des motifs différents en modifiant les modes d'actionnement de la plaque. Par exemple les cellules sont injectées successivement dans la cavité fluidique, à chaque injection le mode d'actionnement est modifié. Les dépôts localisés de cellules réalisés successivement sont possibles car la migration des cellules n'est pas un phénomène instantané.

Par exemple, la plaque est en silicium et les actionneurs sont des actionneurs piézoélectriques en AIN ou PZT. En variante, la plaque est en verre et les actionneurs sont des actionneurs piézoélectriques, par exemple en polymère électroactif, en PZT ou en AIN.

La plaque et les actionneurs sont avantageusement réalisés par des procédés microélectroniques de dépôt et de gravure.

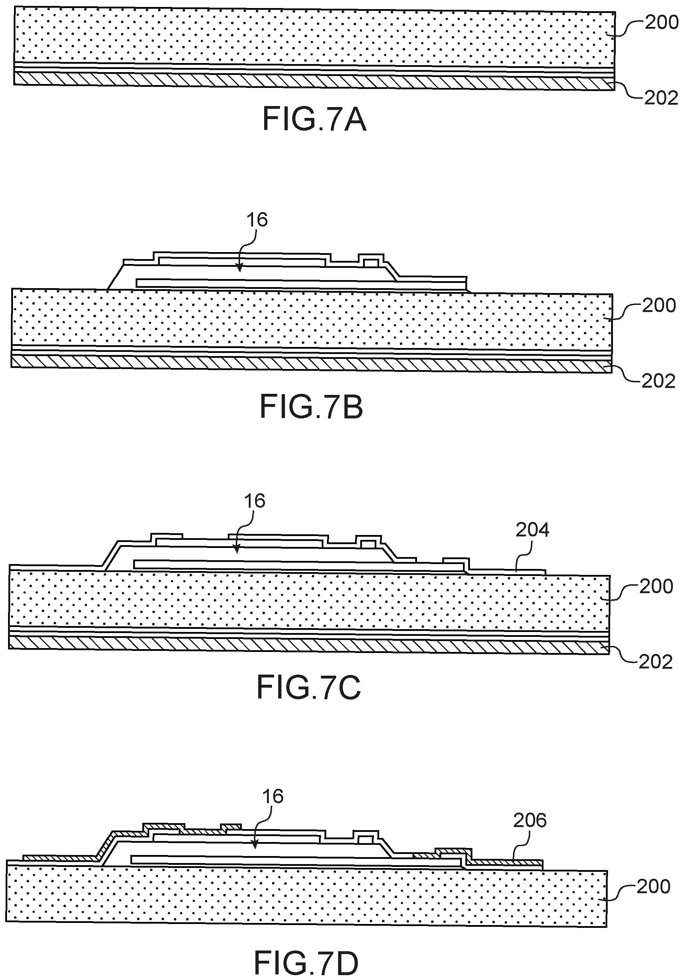

Sur les figures 7A à 7D, on peut voir un exemple de procédé de réalisation d'une plaque en verre portant des actionneurs en AIN, comprenant les étapes qui peuvent être les suivantes :
- Dépôt d'une couche de matériau opaque 202 sur la face arrière du substrat 200. Par exemple le matériau utilisé est du titane. Une couche de titane permet d'opacifier les substrats en verre et rend possible le passage de ces substrats sur des équipements standards de la microélectronique. L'élément ainsi obtenu est représenté sur la figure 7A.
- Formation des actionneurs piézoélectriques 16 sur la face avant, par exemple par dépôt et gravure, les actionneurs comprenant une couche d'AIN entre deux électrodes. L'élément ainsi obtenu est représenté sur la figure 7B.
- Formation d'une couche de passivation 204 sur les actionneurs, par exemple par dépôt. La couche de passivation est ensuite gravée localement pour atteindre les électrodes en vue de formation des contacts électriques. L'élément ainsi obtenu est représenté sur la figure 7C.
- Formation des contacts électriques 206 pour relier les actionneurs à l'unité de commande. Les contacts électriques sont formés en utilisant un masque, en déposant une couche métallique par exemple de l'or, et par gravure de cette couche.
- Retrait de la couche opaque, par exemple par gravure. L'élément ainsi obtenu est représenté sur la figure 7D.

Une couche isolant les contacts du liquide est formée, par exemple il s'agit d'une couche en polymère isolant, par exemple réalisée par dépôt.

La plaque peut ensuite être disposée dans la cavité fluidique, par exemple sur les colonnes et être connectée à l'unité de commande.

Le dispositif selon l'invention permet donc de générer des zones d'exclusion de cellules temporaires adaptées pour l'étude des migrations des cellules.

La localisation ainsi obtenue est maîtrisée et répétable. Elle est indépendante du niveau de technicité de l'opérateur qui, auparavant utilisait un stylet ou un scalpel pour former les cicatrices, et elle ne met pas en œuvre de traitements chimiques qui pourraient influencer la migration des cellules. Le dispositif de localisation est avantageusement compatible avec la culture de cellules de mammifère. Ce système est particulièrement adapté à la quantification de l'impact de molécules thérapeutiques ciblant le cytosquelette pour l'inhibition de l'invasion métastatique dans le cancer.

Selon une autre application, l'invention permet de localiser le développement de bactéries ou de mousses de sorte à laisser transparente la zone de scan des capteurs optiques immergés, ou d'un dispositif de télédétection par laser type LIDAR immergé (Light detection and ranging en terminologie anglo-saxonne).

## Revendications

1. Dispositif de localisation d'objets biologiques de taille donnée comprise entre 1 µm et 100 µm par sédimentation sur une surface d'accueil (8) le dispositif comportant :
- une cavité fluidique (3),
- des moyens de connexion fluidiques (6.1, 6.2) configurés pour permettre d'introduire du liquide et des objets dans la cavité fluidique,
- une plaque (4) munie de la surface d'accueil (8), la surface d'accueil présentant des propriétés d'adhérence vis-à-vis des objets biologiques (C), ladite plaque (4) étant suspendue dans la cavité fluidique (3),
- des moyens (10) pour mettre en vibration la plaque (4) selon au moins un mode stationnaire comportant au moins un actionneur (16) destiné à agir sur la plaque (4) et
- une unité de commande (UC) configurée pour commander le au moins un actionneur de sorte que la plaque vibre selon un mode stationnaire de longueur d'onde λ comprise entre 3 fois et 20 fois la taille donnée des objets biologiques.

2. Dispositif de localisation selon la revendication 1, dans lequel ledit au moins un actionneur (16) est disposé par rapport à la plaque au niveau d'un ventre (V) de la déformée de la plaque en mode stationnaire.

3. Dispositif de localisation selon la revendication 1 ou 2, dans laquelle la plaque (4) est simplement supportée.

4. Dispositif de localisation selon la revendication 3, comportant des supports (14) supportant la plaque (4) suivant une liaison simplement supportée.

5. Dispositif de localisation selon la revendication 4, dans lequel les supports (14) sont en contact avec la plaque (4) au niveau d'au moins certains nœuds (N) de la déformée de la plaque (4) dans ledit mode stationnaire.

6. Dispositif de localisation selon l'une des revendications 1 à 3, dans lequel la plaque est maintenue par encastrement.

7. Dispositif de localisation selon l'une des revendications 1 à 6, dans lequel le au moins un actionneur (16) est un actionneur piézoélectrique fixé à la plaque.

8. Dispositif de localisation selon l'une des revendications 1 à 7, dans lequel au moins une partie de la cavité fluidique (3) est transparente optiquement.

9. Système d'étude de la migration des cellules comportant un dispositif de localisation selon la revendication 8, et des moyens d'observation des objets biologiques sur la plaque.

10. Procédé de localisation d'objets biologiques selon un motif donné mettant en œuvre un dispositif selon l'une des revendications 1 à 8, comportant :
- La mise en place de liquide dans la cavité fluidique (3) au moins sur une partie de la surface d'accueil (8),
- L'injection d'objets biologiques (C) dans ledit liquide,
- L'activation du au moins un actionneur (16) de sorte que la plaque (4) vibre selon un mode stationnaire choisi pour obtenir le motif donné,
- Sédimentation des objets biologiques sur la surface d'accueil (8) selon le motif donné.
- Arrêt du au moins un actionneur pendant ou après la sédimentation.

11. Procédé de localisation selon la revendication 10, dans lequel le liquide entoure la plaque (4).

12. Procédé de localisation selon la revendication 10, dans lequel le liquide est déposé uniquement sur la surface d'accueil (8).

13. Procédé d'analyse de la migration de cellules biologiques mettant en œuvre le procédé de localisation selon l'une des revendications 10 à 12, les objets biologiques étant des cellules, ledit procédé d'analyse comportant, après la sédimentation de cellules biologiques sur la surface d'accueil formant au moins une cicatrice, l'observation de la migration des cellules biologiques.

## Patentansprüche

1. Vorrichtung zur Lokalisierung biologischer Objekte mit einer bestimmten Größe zwischen 1 µm und 100 µm durch Sedimentation auf einer Aufnahmefläche (8), wobei die Vorrichtung Folgendes umfasst:
- einen Fluidhohlraum (3),
- fluidische Verbindungsmittel (6.1, 6.2), die so eingerichtet sind, dass sie das Einführen von Flüssigkeit und Gegenständen in den Fluidhohlraum ermöglichen,
- eine Platte (4), die mit der Aufnahmefläche (8) versehen ist, wobei die Aufnahmefläche Hafteigenschaften gegenüber biologischen Objekten (C) aufweist, wobei die Platte (4) in dem Fluidhohlraum (3) aufgehängt ist,
- Mittel (10) zum Vibrieren der Platte (4) nach mindestens einem stationären Modus umfassend mindestens ein Stellglied (16), das auf die Platte (4) einwirken soll, und
- eine Steuereinheit (UC), die so konfiguriert ist, dass sie das mindestens eine Stellglied so steuert, dass die Platte in einem stationären Wellenlängenmodus zwischen dem λ 3-fachen und 20-fachen der gegebenen Größe der biologischen Objekte vibriert.

2. Vorrichtung zur Lokalisierung nach Anspruch 1, wobei das mindestens eine Stellglied (16) in Bezug auf die Platte an einem Bauchstück (V) der Verformung der Platte im stationären Modus angeordnet ist.

3. Vorrichtung zur Lokalisierung nach Anspruch 1 oder 2, wobei die Platte (4) einfach getragen wird.

4. Vorrichtung zur Lokalisierung nach Anspruch 3, umfassend Halterungen (14), die die Platte (4) gemäß einer einfach getragenen Verbindung tragen.

5. Vorrichtung zur Lokalisierung nach Anspruch 4, wobei die Halterungen (14) an mindestens einigen Knoten (N) der verformten Platte (4) im besagten stationären Modus Kontakt mit der Platte (4) haben.

6. Vorrichtung zur Lokalisierung nach einem der Ansprüche 1 bis 3, wobei die Platte durch Einbettung gehalten wird.

7. Vorrichtung zur Lokalisierung nach einem der Ansprüche 1 bis 6, wobei das mindestens eine Stellglied (16) ein an der Platte befestigtes piezoelektrisches Stellglied ist.

8. Vorrichtung zur Lokalisierung nach einem der Ansprüche 1 bis 7, wobei mindestens ein Teil des Fluidhohlraumes (3) optisch transparent ist.

9. System zur Untersuchung der Zellmigration umfassend eine Vorrichtung zur Lokalisierung nach Anspruch 8 und Mittel zur Beobachtung biologischer Objekte auf der Platte.

10. Verfahren zur Lokalisierung biologischer Objekte nach einem bestimmten Muster zur Umsetzung einer Vorrichtung nach einem der Ansprüche 1 bis 8, umfassend:
- Einbringen von Flüssigkeit in den Fluidhohlraum (3) auf mindestens einem Teil der Aufnahmefläche (8),
- Injektion von biologischen Objekten (C) in die Flüssigkeit,
- Aktivierung des mindestens einen Stellglieds (16), sodass die Platte (4) in einem stationären Modus vibriert, der gewählt wurde, um das vorgegebene Muster zu erhalten,
- Sedimentation der biologischen Objekte auf der Aufnahmefläche (8) nach vorgegebenem Muster.
- Abschaltung von mindestens einem Stellglied während oder nach der Sedimentation.

11. Verfahren zur Lokalisierung nach Anspruch 10, wobei die Flüssigkeit die Platte (4) umgibt.

12. Verfahren zur Lokalisierung nach Anspruch 10, wobei die Flüssigkeit nur auf der Aufnahmefläche (8) abgelagert wird.

13. Verfahren zur Analyse der Migration biologischer Zellen, das das Verfahren zur Lokalisierung nach einem der Ansprüche 10 bis 12 umsetzt, wobei die biologischen Objekte Zellen sind, wobei das Verfahren zur Analyse nach der Sedimentation biologischer Zellen auf der Aufnahmefläche, die mindestens eine Narbe bildet, die Beobachtung der Migration biologischer Zellen umfasst.

## Claims

1. Device for locating biological objects of at least a given size between 1 µm and 100 µm on a accommodating surface (8), including:
- a fluid cavity (3),
- fluid connection means (6.1, 6.2) configured to make to introduce liquid and biological objects into the fluid cavity,
- a plate (4) provided with said accommodating surface (8) having properties of adhesion vis-à-vis biological objects (C), said plate (4) being suspended in the fluid cavity (3),
- means (10) for making the plate (4) vibrate in at least one stationary mode, said at least one actuator (16) being configured to act on the plate (4), and
- a control unit (UC) configured to control the at least one actuator so that the plate vibrates in a stationary mode with a wavelength λ of between 3 times and 20 times the given size of the biological objects.

2. Location device according to claim 1, wherein said at least one actuator (16) is disposed with respect to the plate at an antinode (V) of the deflected shape of the plate in stationary mode.

3. Location device according to any of the claims 1 or 2, wherein the plate (4) is simply supported.

4. Location device according to claim 3, including supports (14) supporting the plate (4) in a simply supported connection.

5. Location device according to claim 4, wherein the supports (14) are in contact with the plate (4) at least some nodes (N) of the deflected shape of the plate (4) in said stationary mode.

6. Location device according to any of the claims 1 to 3, wherein the plate is held by embedding.

7. Location device according to any of the claims 1 to 6, wherein the at least one actuator (16) is a piezoelectric actuator fixed to the plate.

8. Location device according to any of the claims 1 to 7, wherein at least part of the fluid cavity is optically transparent.

9. System for studying the migration of cells including a location device according to claim 8, and a device for observing the biological objects on the plate.

10. Method for locating biological objects in a given pattern using a device according to any of the claims 1 to 8, including:
- the placing of liquid in the fluid cavity (3) at least over part of the accommodating surface (8),
- the injection of biological objects (C) into said liquid,
- the activation of the at least one actuator (16) so that the plate (4) vibrates in a stationary mode chosen so as to obtain the given pattern,
- sedimentation of the biological objects on the accommodating surface (8) in the given pattern,
- stoppage of the at least one actuator during or after the sedimentation.

11. Location method according to claim 10, wherein the liquid surrounds the plate (4).

12. Location method according to claim 10, wherein the liquid is deposited solely on the accommodating surface (8).

13. Method for analyzing the migration of biological cells using the location method according to any of the claims 10 to 12, the biological objects being cells, said analysis method including, after the sedimentation of biological cells on the accommodating surface forming at least one scar, the observation of the migration of the biological cells.
